# EUROPÄISCHE PATENTSCHRIFT

(11) **EP 2 238 920 B1**
(45) Veröffentlichungstag und Bekanntmachung des Hinweises auf die Patenterteilung: **20.02.2013**
(21) Anmeldenummer: 09005189.7
(22) Anmeldetag: 09.04.2009
(51) Int. Cl.: A61B 17/16

(54) **Medizinisches, insbesondere chirurgisches, Handstück**
Medical, in particular surgical, handpiece
Pièce à main médicale, en particulier chirurgicale

(43) Veröffentlichungstag der Anmeldung: 13.10.2010
(73) Patentinhaber: Arthrex Inc, Naples, FL 34108-1945 (US)
(72) Erfinder: Kainhofer, Manfred, 5071 Wals-Siezenheim (AT); Schmied, Walter, 5121 Ostermiething (AT)
(74) Vertreter: Lohr, Georg

(56) Entgegenhaltungen:
- DE-A1- 3 639 696

## Beschreibung

Die vorliegende Erfindung bezieht sich auf ein medizinisches, insbesondere chirurgisches, Handstück zum Antreiben eines medizinischen Werkzeugs nach dem Oberbegriff des Anspruchs 1.

Derartige medizinische, insbesondere chirurgische, Handstücke dienen zum Antrieb medizinischer Werkzeuge wie zum Beispiel Sägen, Bohrern oder chirurgischer Bohrdrähte. Hierzu werden die Werkzeuge über Kupplungsvorrichtungen mit dem Handstück verbunden. Somit kann mittels eines Handstücks eine Vielzahl von verschiedenen Werkzeugen betrieben werden.

Chirurgische Bohrdrähte dienen in der Chirurgie zur Frakturbehandlung. Hierbei werden die Bohrdrähte, vorzugsweise mittels eines angetriebenen Handstücks, in Rotation versetzt und in das Knochenmaterial eingebohrt. Durch mehrmaliges Wiederholen des Ablaufs "Einbohren in das Knochenmaterial, Lösen der Spannvorrichtung, Zurücksetzen des Handstücks, erneutes Spannen des Drahtes und Einbohren" kann so der Bohrdraht mittels des Handstücks leicht und ohne erheblichen Kraftaufwand in das Knochenmaterial eingebracht werden.

Um die Länge der chirurgischen Bohrdrähte unabhängig von den angetriebenen Handstücken, insbesondere der den Draht aufnehmenden Spannvorrichtungen, ausbilden zu können, werden die für diese Anwendung vorgesehenen Handstücke mit Durchgangsbohrungen für den Draht versehen, die sich durch das Innere des Handstücks erstrecken. Somit können Bohrdrähte mit beliebiger Länge in der Spannvorrichtung aufgenommen und mittels eines Antriebs, vorzugsweise rotierend oder oszillierend, angetrieben werden.

Ein derartiges medizinisches, insbesondere chirurgisches, Handstück zur Aufnahme eines chirurgischen Bohrdrahtes im Inneren des Handstücks, insbesondere in dessen Antriebsstrang, ist aus der US 5,207,697 bekannt.

Dieses bekannte chirurgische Handstück zum Einbringen von Bohrdrähten in Knochenmaterial weist ein Handstückgehäuse mit einem darin aufgenommenen Elektroantrieb mit einer ersten Antriebswelle und ersten Antriebsdrehzahl auf. Diese erste Welle ist über ein Getriebe mit einer zweiten, koaxial zur ersten Welle angeordneten Ausgangswelle verbunden. Diese mit einer zweiten geringeren Ausgangsdrehzahl rotierende Welle dient zum Antrieb einer auf das Handstück aufsetzbaren Kupplungsvorrichtung für ein chirurgisches Werkzeug, insbesondere für einen chirurgischen Bohrdraht. Auf der gegenüberliegenden Seite der Aufnahmeöffnung für die Kupplungsvorrichtung befindet sich am Handstück eine zweite Gehäuseöffnung koaxial zu den zwei Wellen des Antriebstangs zum Einführen eines Bohrdrahtes durch die Wellenanordnung hindurch zur Aufnahme in der Kupplungsvorrichtung. Auf Grund der unterschiedlichen Drehzahlen der mehreren Wellen des Antriebstangs kam es bislang zum Abrieb bis hin zum Abschnüren des Bohrdrahtes. Um nun diesen Abrieb am Bohrdraht zu vermeiden, wird in der vorliegenden Schrift die Ausgangswelle mittels einer dritten Welle, welche sich durch die Antriebswelle hindurch erstreckt, verlängert. Somit wird gewährleistet, dass auf den Bohrdraht durch das gesamte Handstück hindurch nur eine, nämlich die Ausgangsdrehzahl der miteinander verbundenen zweiten und dritten Welle, wirkt.

Ein weiteres medizinisches, insbesondere chirurgisches, Handstück zur Aufnahme eines chirurgischen Drahtes ist aus der DE 3639696 A1 bekannt.

Das bekannte Handstück weist ein Gehäuse mit einem darin angeordneten Elektromotor mit einer Motorwelle auf. Die Motorwelle treibt über eine erste Getriebestufe, nämlich über einen Riemen, eine Abtriebswelle an. Diese zweite hohle Welle ist wiederrum über ein zweites Getriebe mit einem hohlen Wellenstumpf, welcher als Träger für ein Spannfutter dient, verbunden. Mit dem Wellenstumpf ist ein hohles Führungsrohr verbunden, welches sich durch die Abtriebswelle erstreckt. Hierdurch wird gewährleistet, dass auf den Bohrdraht nur eine Drehzahl, nämlich die Ausgangsdrehzahl des Wellenstumpfs, wirkt.

Als nachteilig dieser Ausgestaltungen der Handstück zum Antreiben eines medizinischen Werkzeuges, insbesondere eines chirurgischen Bohrdrahtes, erweist sich die Abhängigkeit der auf den Draht wirkenden Drehzahl von der Wellenanordnung des Antriebstangs. Die Drehzahl der den Bohrdraht durch das Handstück führenden Wellenanordnung, nämlich der miteinander verbundenen zweiten und dritten Welle, wird durch den im Handstück befindlichen Motor und dessen Getriebe bestimmt. Werden wie bereits erwähnt unterschiedliche Werkzeuge mit dem Handstück verbunden, sind hierzu unterschiedliche Antriebsdrehzahlen nötig. Hierzu weisen die jeweilig zu den Werkzeugen gehörenden Kupplungsvorrichtungen weitere Getriebeanordnungen auf, um die Ausgangsdrehzahl des Handstücks auf die vorgeschriebenen Antriebsdrehzahlen der Werkzeuge anzupassen. In Folge dessen tritt eine weitere, nicht von dem Stand der Technik berücksichtigte, Drehzahldifferenz zwischen der Ausgangsdrehzahl des Handstücks und der Antriebsdrehzahl der Kupplungsvorrichtung auf. Dies führt erneut zu einer Beschädigung des Bohrdrahtes.

Einen weiteren Nachteil der im Stand der Technik bekannten Ausführungsformen stellt die fehlende Lagerung der dritten Welle dar. Durch die Anordnung der dritten bzw. verlängerten zweiten Welle in der Antriebswelle ohne zusätzlicher Lagerung gegenüber der Antriebswelle kommt es lediglich zu einer Verlagerung des Abriebs am Bohrdraht zu einem Abrieb an der dritten Welle. Durch das Einführen des Bohrdrahtes durch die Wellenanordnung, insbesondere durch die dritte Welle, in die Kupplungsvorrichtung, sowie beim Zurücksetzen des Handstücks beim Einbohren des Drahtes in das Knochenmaterial, als auch auf Grund der eigenen Unwucht es Drahtes im angetriebenen Zustand, ist ein Kontakt der ineinander angeordneten und mit zwei unterschiedlichen Drehzahlen betriebenen ersten und dritten Welle ohne gesonderter Lagerung der dritten Welle nicht vermeidbar. Dies führt schließlich zu einem erhöhten Verschleiß der dritten Welle.

Der vorliegenden Erfindung liegt daher die Aufgabe zugrunde, ein medizinisches, insbesondere chirurgisches, Handstück zu schaffen, welches die Aufnahme eines Werkzeugs, insbesondere eines Bohrdrahts, in dem Antriebsstrang eines Handstücks ermöglicht, ohne den Bohrdraht auf Grund auftretender Drehzahldifferenzen innerhalb des Antriebstanges zu beschädigen. Des Weiteren soll eine handstückunabhängige, insbesondere drehzahlunabhängige, Aufnahme eines Werkzeugs in einem Handstück, insbesondere in dessen Antriebsstrang, ermöglicht werden.

Erfindungsgemäß wird diese Aufgabe durch ein medizinisches, insbesondere chirurgisches, Handstück gemäß Anspruch 1 gelöst.

In dem vorliegenden medizinischen, insbesondere chirurgischen, Handstück ist ein Antriebsstrang mit einem Motor, vorzugsweise einem Elektromotor, mit einer Antriebswelle angeordnet. Zur Aufnahme beliebig langer Bohrdrähte in einer an das Handstück anschließbaren oder fest verbundenen Kupplungsvorrichtung weisen zumindest Jeilbereiche des Antriebsstrangs eine das Handstück durchsetzende Durchgangsbohrung auf. Diese ist vorzugsweise durch zumindest eine hülsenförmige Welle im Antriebsstrang sowie durch eine koaxial zur Antriebswelle angeordnete Gehäuseöffnung an der Rückseite des Handstücks ausgebildet. Zur Aufnahme und Durchführung von Bohrdrähten durch die sich drehende Wellenanordnung, ohne Beschädigungen am Draht hervorzurufen, weist der vorliegende Antriebsstrang eine zweite sich relativ zur ersten Antriebswelle frei drehbar gelagerte zweite Welle auf, welche unabhängig von der ersten Welle rotierbar ist. Durch die Lagerung der zweiten Welle mittels zumindest eines Lagers gegenüber dem Antriebsstrang, insbesondere gegenüber einer Antriebswelle, oder einem drehfesten Bauteil des Handstücks wird die zweite Welle antriebslos in dem Antriebsstrang aufgenommen und nicht von dem Motor und/ oder dessen Getriebeeinheit angetrieben und kann somit frei gegenüber der Antriebswelle rotieren.

Bei Anordnung einer Getriebeeinheit in dem Antriebsstrang des Handstücks ist die Antriebswelle vorzugsweise zweiteilig als Motorwelle und erste Ausgangswelle ausgebildet. Zusätzlich kann eine zweite Ausgangswelle über das Getriebe von der Antriebswelle angetrieben werden. Um nun ein Werkzeug unabhängig von der Drehzahl des Handstücks im Antriebsstrang aufzunehmen, erstreckt sich die zweite frei drehbar gelagerte Welle durch die Motorwelle und erste Ausgangswelle und/ oder der zweiten Ausgangswelle.

Zur Lagerung der zweiten Welle frei drehbar in dem Antriebsstrang wird die Welle mittels zumindest eines Lagers, insbesondere eines Wälzlagers, an einem Rücksprung einer Wellenwand einer hülsenförmigen Welle des Antriebstrangs oder einem drehfesten Bauteil des Handstücks, insbesondere an einer koaxial zum Antriebsstrang angeordneten Gehäuseöffnung, gegengelagert.

Zur Übertragung der Antriebsbewegung von dem Motor des Handstücks auf den Bohrdraht weist das Handstück des Weiteren eine an den Antriebsstrang, insbesondere an die erste oder zweite Ausgangswelle, anschließbare oder fest verbundene Kupplungsvorrichtung mit Kupplungswelle auf. Diese kann mit einer zweiten Getriebeeinheit ausgebildet sein, um die Ausgangsdrehzahl des Handstücks auf die Antriebsdrehzahl des jeweiligen Werkzeugs anzupassen. Bei einer fest mit dem Antriebsstrang verbundenen Kupplungsvorrichtung kann sich die zweite den Bohrdraht aufnehmende Welle zumindest teilweise durch die Kupplungswelle erstrecken. Bei einer von dem Antriebsstrang lösbaren Kupplungsvorrichtung ist die zweite Welle vorzugsweise mehrteilig, insbesondere zweiteilig, zur Lagerung in der Antriebswelle und/ oder Ausgangswelle sowie in der Kupplungswelle ausgebildet.

Das vorliegende medizinische, insbesondere chirurgische, Handstück zeichnet sich durch eine Reihe erheblicher Vorteile aus.

Durch die Lagerung einer relativ zu einer ersten Antriebswelle eines Antriebstangs frei drehbar gelagerten zweiten Welle wird ein Handstück, insbesondere für Bohrdrähte, geschaffen, das eine Aufnahme und das Hindurchführen eines Bohrdrahtes durch die Wellenanordnung des Handstücks unabhängig von der Drehzahl des Antriebsstrangs ermöglicht. Durch die drehzahlunabhängige Durchführung des Handstücks wird das Ankuppeln verschiedenster Kupplungsvorrichtungen mit jeweils unterschiedlichen Antriebsdrehzahlen gewährleistet, ohne eine Drehzahldifferenz zwischen Ausgangsdrehzahl des Handstücks und Antriebsdrehzahl einer, vorzugsweise lösbaren, Kupplungsvorrichtung zu erzeugen.

Einen weiteren Vorteil der Erfindung bildet die Lagerung der zweiten Welle gegenüber der Antriebswelle mittels zumindest eines Lagers, vorzugsweise mittels eines Wälzlagers. Durch die Lagerung innerhalb der Antriebswelle und/ oder gegenüber einem drehfesten Bauteil des Handstücks, wird ein Kontakt der zweiten Welle mit der Antriebswelle verhindert, der zum Beispiel durch das Einführen des Bohrdrahtes durch die zweite Welle in die Kupplungsvorrichtung, sowie beim Zurücksetzen des Handstücks beim Einbohren des Drahtes in das Knochenmaterial, als auch auf Grund der eigenen Unwucht des Drahtes im angetriebenen Zustand, entstehen kann. Somit wird ein Verschleiß der Wellen, auf Grund möglicher Drehzahldifferenzen und der dadurch entstehenden Reibung, vermieden.

Im Rahmen der Erfindung versteht es sich selbstverständlich, dass das medizinische, insbesondere chirurgische, Handstück nicht auf Handstücke zur Durchführung von chirurgischen Bohrdrähten beschränkt ist. Das Handstück kann mit einer Vielzahl von medizinischen Werkzeugen verwendet werden.

Nachfolgend wird die Erfindung anhand mehrerer Ausführungsbeispiele und in Verbindung mit den beigefügten Zeichnungen erläutert.

Dabei zeigt:
Figur 1 einen Längsschnitt durch das medizinische, insbesondere chirurgische, Handstück,
Figur 2 einen Längsschnitt durch eine erste Ausführungsform des Antriebsstrangs des medizinischen, insbesondere chirurgischen Handstücks,
Figur 3 einen Längsschnitt durch eine zweite Ausführungsform des Antriebsstrangs des medizinischen, insbesondere chirurgischen Handstücks,
Figur 4 einen Querschnitt durch das medizinische, insbesondere chirurgische, Handstück;

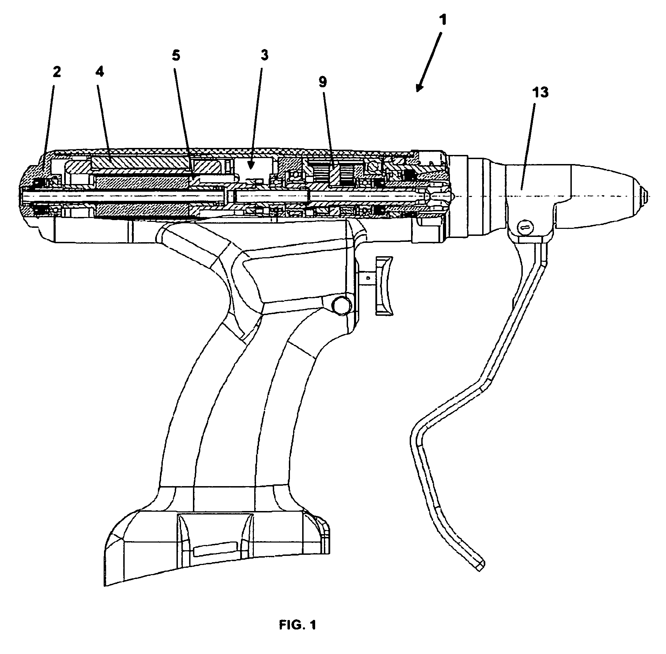

Das in Figur 1 dargestellte medizinische, insbesondere chirurgische, Handstück 1 ist als pistolenförmiges Instrument mit einer daran anschließbaren oder festverbundenen Kupplungsvorrichtung 13 ausgebildet. In dem Gehäuse 2 des Handstücks 1 ist ein Antriebsstrang 3 zum Antreiben eines Werkzeugs mittels der Kupplungsvorrichtung 13 angeordnet. Der Antriebsstrang 3 setzt sich zumindest aus einem Motor 4, insbesondere einem Elektromotor oder einem Luftmotor, und einer ersten Antriebswelle 5 zusammen. Des Weiteren kann der Antriebsstrang 3 ein Getriebe 9 aufweisen.

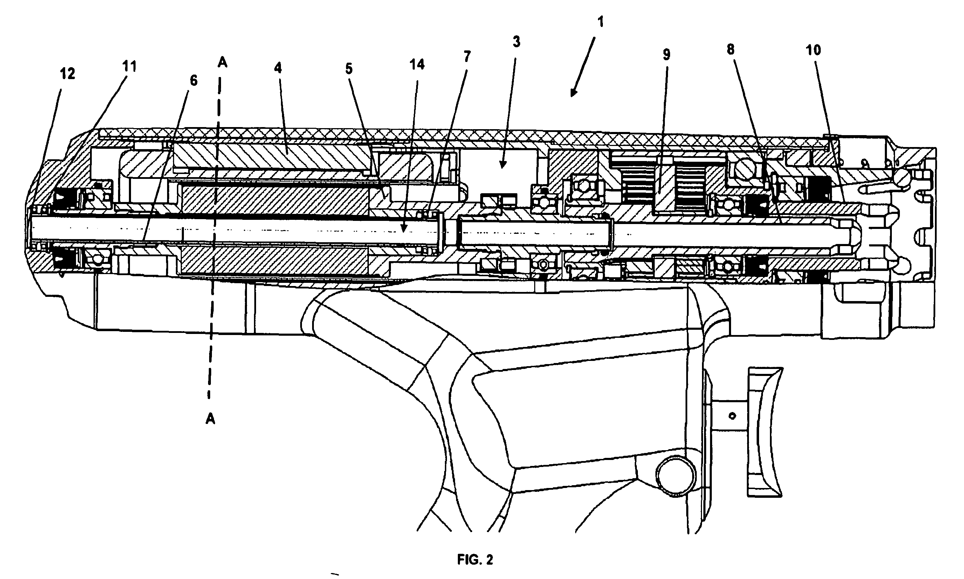

In Figur 2 ist der Antriebsstrang 3 in dem Gehäuse 2 des Handstücks 1 abgebildet. Der Motor 4 und dessen Motorwelle 5 treiben eine erste Ausgangswelle 8 sowie über ein Getriebe 9 eine zweite Ausgangswelle 10 an. Zur Aufnahme und Durchführung eines beliebig langen Bohrdrahtes durch den Antriebsstrang 3 des Handstücks 1, ohne diesen auf Grund möglicher Drehzahldifferenzen der mehreren Wellen zu beschädigen, ist eine zweite Welle 6 relativ zur ersten Antriebswelle 5 mittels der Lager 7 und 11 frei drehbar gelagert, so dass die zweite Welle 6 unabhängig von der ersten Welle 5 rotierbar ist. Am rückseitigen Ende des Handstücks 1 befindet sich eine Gehäuseöffnung 12 koaxial zur hülsenförmigen Wellenanordnung des Antriebstangs 3 und bildet mit dieser eine das Handstück 1 durchsetzende Durchgangsbohrung 14. Die Lager 7 und 11 sind insbesondere als Wälzlager, vorzugsweise als Kugellager, ausgebildet und im Antriebsstrang 3 oder an einem drehfesten Bauteil des Handstücks 1 gegengelagert. Hierzu sind die Lager 7 und 11 vorzugsweise an einem Ende des Antriebsstrangs 3, oder eines Wellenteils des Antriebsstrangs 3, vorzugsweise mittels eines Rücksprungs in der Wellenwand, oder in einer koaxial zum Antriebsstrang 3 angeordneten Gehäuseöffnung 12 aufgenommen.

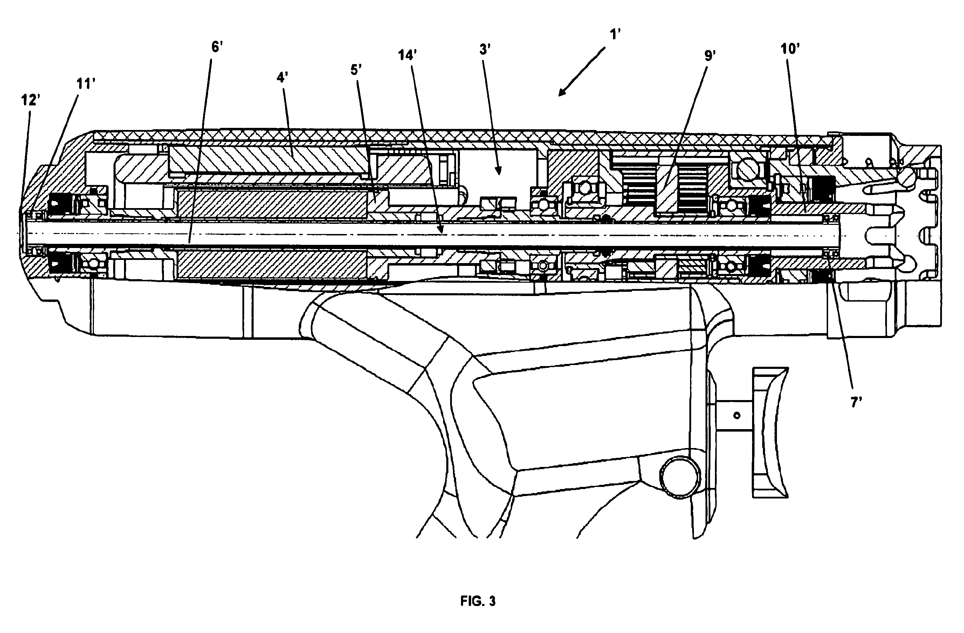

In Figur 3 ist eine alternative Ausführungsform des Antriebsstrangs sowie der Anordnung und Lagerung der zweiten Welle in dem Handstücks gezeigt. Mittels des Motors 4' wird in dem Antriebsstrang 3' eine Motorwelle 5' angetrieben. Diese Welle 5' treibt wiederum über ein Getriebe 9' eine Ausgangswelle 10' an. Die zweite Welle 6' erstreckt sich bei dieser Ausführung des Antriebstrangs 3' durch die Antriebswelle 5' und Ausgangswelle 10'. Durch die Lagerung der zweiten Welle 6' mittels des Lagers 7' gegen einen Rücksprung in der Wellenwand der Ausgangswelle 10' und des Lagers 11' gegenüber einem drehfesten Bauteil des Handstücks 1' wird die zweite Welle 6' relativ zur Antriebswelle 5' und Ausgangswelle 10' frei drehbar gelagert, so dass die zweite Welle 6' unabhängig von der Antriebswelle 5' und Ausgangswelle 10' rotierbar ist. Die das Handstück 1' durchsetzende Durchgangsbohrung 14' und die Gehäuseöffnung 12' ermöglichen somit eine handstückunabhängige, insbesondere drehzahlunabhängige, Aufnahme und Durchführung eines Bohrdrahtes durch den Antriebsstrang 3'.

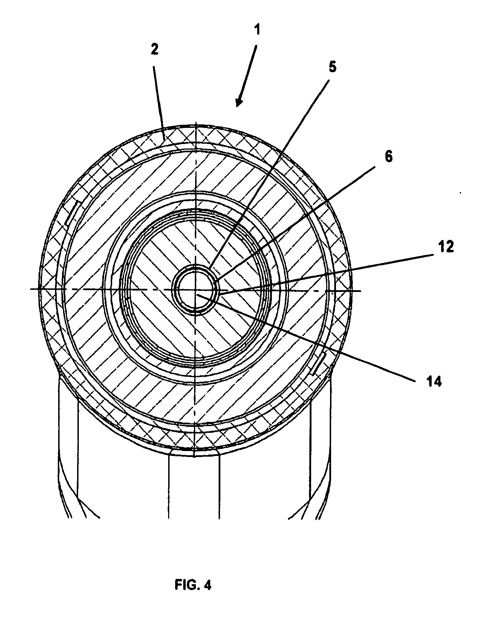

In Figur 4 ist ein Querschnitt durch das Handstück 1 gemäß Figur 2 entlang der Linie A-A gezeigt. In dem Gehäuse 2 des Handstücks 1 ist ein Antriebsstrang mit dessen Antriebswelle 5 angeordnet. In der Antriebswelle 5 ist die zweite frei drehbar gelagerte Welle 6 gelagert. Diese insbesondere hülsenförmige Welle bildet zusammen mit der Gehäuseöffnung 12 eine zentrale Durchgangsbohrung 14 für ein Werkzeug, insbesondere für einen Bohrdraht, durch das Handstück 1.

Die Erfindung ist nicht auf die beschriebenen Ausführungsbeispiele beschränkt. Im Rahmen der Erfindung versteht es sich selbstverständlich, dass die Durchgangsbohrung für das Werkzeug, insbesondere für den Bohrdraht, nicht das gesamte Handstück durchsetzen muss. Bei Verwendung von Drähten deren Längen nicht die Länge des Antriebsstrangs überschreiten, ist es möglich nur Teilbereiche des Antriebsstrangs mit einer Durchgangsbohrung zu versehen. Des Weiteren kann die Lagerung der antriebslosen zweiten Welle mittels verschiedenster Formen von Lagern, wie zum Beispiel Luftlager oder Gleitlager, erfolgen.

## Patentansprüche

1. Medizinisches, insbesondere chirurgisches, Handstück (1, 1') zum Antreiben eines medizinischen Werkzeugs mit einem Gehäuse (2, 2'), einem darin aufgenommenen Antriebsstrang (3, 3'), welcher einen Motor (4, 4') mit einer ersten Antriebswelle (5, 5') aufweist, wobei zumindest Teilbereiche des Antriebsstrangs (3, 3') mit einer Durchgangsbohrung (14, 14') für das Werkzeug versehen sind, einer an den Antriebsstrang (3, 3') anschließbaren oder damit fest verbundenen Kupplungsvorrichtung (13) zur Aufnahme des Werkzeugs mit einer Kupplungswelle, sowie einer zweiten, hülsenförmigen Welle (6, 6'), wobei die zweite Welle zumindest teilweise in der ersten Antriebswelle (5, 5') angeordnet ist, **dadurch gekennzeichnet, dass** die zweite Welle (6, 6') relativ zur ersten Antriebswelle (5, 5') mittels zumindest eines Lagers (7, 7') frei drehbar gelagert ist, so dass die zweite Welle (6, 6') unabhängig von der ersten Antriebswelle (5,5') rotierbar ist.

2. Medizinisches, insbesondere chirurgisches, Handstück (1, 1') nach Anspruch 1, **dadurch gekennzeichnet, dass** die erste Antriebswelle (5, 5') mehrteilig, vorzugsweise zweiteilig, als Motorwelle und erste Ausgangswelle (8), ausgeführt ist.

3. Medizinisches, insbesondere chirurgisches, Handstück (1, 1') nach Anspruch 1 oder 2, **dadurch gekennzeichnet, dass** der Antriebsstrang (3, 3') des Weiteren ein Getriebe (9, 9') mit einer zweiten Ausgangswelle (10, 10') aufweist, wobei das Getriebe mit der ersten Antriebswelle (5, 5') verbunden ist, um die zweite Ausgangswelle (10, 10') mit einer von der ersten Ausgangswelle (5, 5') unterschiedlichen Drehzahl anzutreiben.

4. Medizinisches, insbesondere chirurgisches, Handstück (1, 1') nach Anspruch 2 oder 3, **dadurch gekennzeichnet, dass** sich die zweite Welle (6, 6') zumindest teilweise durch die zweite Ausgangswelle (10, 10') erstreckt.

5. Medizinisches, insbesondere chirurgisches, Handstück (1, 1') nach einem der vorherigen Ansprüche, **dadurch gekennzeichnet, dass** das zumindest eine zur Lagerung der zweiten Welle (6, 6') dienende Lager (7, 7') im Antriebsstrang aufgenommen ist.

6. Medizinisches, insbesondere chirurgisches, Handstück (1, 1') nach Anspruch 5, **dadurch gekennzeichnet, dass** das zur Lagerung der zweiten Welle (6, 6') dienende Lager (7, 7') an einem Ende des Antriebsstrangs, oder eines Wellenteils des Antriebsstrangs, vorzugsweise mittels eines Rücksprungs in der Wellenwand, gegengelagert ist.

7. Medizinisches, insbesondere chirurgisches, Handstück (1, 1') nach einem der vorherigen Ansprüche, **dadurch gekennzeichnet, dass** das zumindest eine zur Lagerung der zweiten Welle (6, 6') dienende Lager (11, 11') an einem drehfesten Bauteil des Handstücks (1, 1') gelagert ist.

8. Medizinisches, insbesondere chirurgisches, Handstück (1, 1') nach Anspruch 7, **dadurch gekennzeichnet, dass** das zur Lagerung der zweiten Welle (6, 6') dienende Lager (11, 11') in einer koaxial zum Antriebsstrang angeordneten Gehäuseöffnung (12, 12') aufgenommen ist.

9. Medizinisches, insbesondere chirurgisches, Handstück (1, 1') nach einem der vorherigen Ansprüche, **dadurch gekennzeichnet, dass** das zumindest eine Lager (7, 7'; 11, 11') ein Wälzlager, insbesondere ein Kugellager, ist.

10. Medizinisches, insbesondere chirurgisches, Handstück (1, 1') nach einem der vorherigen Ansprüche, **dadurch gekennzeichnet, dass** die Kupplungsvorrichtung (13) ein zweites Getriebe aufweist.

11. Medizinisches, insbesondere chirurgisches, Handstück (1, 1') nach einem der vorherigen Ansprüche, **dadurch gekennzeichnet, dass** sich die zweite Welle (6, 6') zumindest teilweise durch die Kupplungswelle (13) erstreckt.

12. Medizinisches, insbesondere chirurgisches, Handstück (1, 1') nach einem der vorherigen Ansprüche, **dadurch gekennzeichnet, dass** die zweite Welle (6, 6') mehrteilig, vorzugsweise zweiteilig, zur Lagerung in der Ausgangswelle (8; 10, 10') sowie in der Kupplungswelle, ausgebildet ist.

13. Medizinisches, insbesondere chirurgisches, Handstück (1, 1') nach einem der vorherigen Ansprüche, **dadurch gekennzeichnet, dass** der Antriebsstrang (3, 3') und die zweite Welle (6, 6') eine das Handstück durchsetzende Durchgangsbohrung (14, 14') aufweisen.

## Claims

1. Medical, in particular a surgical, handheld medical-tool drive unit (1, 1'), comprising a housing (2, 2'), a drive train (3, 3') accommodated therein and having a motor (4, 4') with a first drive shaft (5, 5'), at least portions of the drive train (3, 3') being provided with a through bore (14, 14') for the tool, a coupling device (13) for accommodating the tool, the coupling device being connectable or firmly connected to the drive train (3, 3') and having a coupling shaft, and also a second, sleeve-shaped shaft (6, 6'), with the second shaft being at least partially disposed within the first drive shaft (5, 5'), **characterized in that** the second shaft (6, 6') is supported to be freely rotatable relative to the first drive shaft (5, 5') by means of at least one bearing (7, 7'), so that the second shaft (6, 6') is adapted to be rotated independently from the first drive shaft (5, 5').

2. Medical, in particular a surgical, handheld unit (1, 1') according to claim 1, **characterized in that** the first drive shaft (5, 5') is designed to be of multiple parts, preferably two parts, as motor shaft, and first output shaft (8).

3. Medical, in particular a surgical, handheld unit (1, 1') according to claim 1 or 2, **characterized in that** the drive train (3, 3') furthermore has a gear (9,9') with a second output shaft (10, 10'), with the gear being connected to the first drive shaft (5, 5') to drive the second output shaft (10, 10') at a rotation number different from that of the first output shaft (5, 5').

4. Medical, in particular a surgical, handheld unit (1, 1') according to claim 2 or 3, **characterized in that** the second shaft (6, 6') at least partially extends through the second output shaft (10, 10').

5. Medical, in particular a surgical, handheld unit (1, 1') according to any one of the preceding claims, **characterized in that** the at least one bearing (7. 7') serving to support the second shaft (6, 6') is accommodated in the drive train.

6. Medical, in particular a surgical, handheld unit (1, 1') according to claim 5, **characterized in that** the bearing (7, 7') serving to support the second shaft (6. 6') is counter-supported at one end of the drive train or of a shaft portion of the drive train, preferably by means of a recess in the shaft wall.

7. Medical, in particular a surgical, handheld unit (1, 1') according to any one of the preceding claims, **characterized in that** the at least one bearing (11, 11') serving to support the second shaft (6, 6') is supported at a co-rotating component of the handheld unit (1, 1').

8. Medical, in particular a surgical, handheld unit (1, 1') according to claim 7, **characterized in that** the bearing (11, 11') serving to support the second shaft (6, 6') is accommodated in a housing opening (12, 12') disposed coaxially with the drive chain.

9. Medical, in particular a surgical, handheld unit (1, 1') according to any one of the preceding claims, **characterized in that** the at least one bearing (7, 7'; 11, 11') is a rolling bearing, in particular a ball bearing.

10. Medical, in particular a surgical, handheld unit (1, 1') according to any one of the preceding claims, **characterized in that** the coupling device (13) comprises a second gearing.

11. Medical, in particular a surgical, handheld unit (1, 1') according to any one of the preceding claims, **characterized in that** the second shaft (6, 6') extends at least partially through the coupling shaft (13).

12. Medical, in particular a surgical, handheld unit (1, 1') according to any one of the preceding claims, **characterized in that** the second shaft (6, 6') is of multipart, preferably two-part, design for being supported in the output shaft (8; 10, 10') and also in the coupling shaft .

13. Medical, in particular a surgical, handheld unit (1, 1') according to any one of the previous claims, **characterized in that** the drive chain (3, 3') and the second shaft (6, 6') comprise a through bore (14, 14') passing through the handheld unit.

## Revendications

1. Poignée (1, 1') à usage médical, en particulier chirurgical, pour l'entraînement d'un outil médical, avec un corps (2, 2'), une chaîne de transmission (3, 3') logée dans celle-ci qui comprend un moteur (4, 4') avec un premier arbre d'entraînement (5, 5'), des parties au moins de la chaîne de transmission (3, 3') étant munies d'un alésage traversant (14, 14') pour l'outil, avec un dispositif de couplage (13) pour recevoir l'outil pouvant être raccordé à la chaîne de transmission (3, 3') ou relié à demeure à celle-ci et muni d'un arbre d'accouplement, et avec un deuxième arbre (6, 6') en forme de manchon, lequel deuxième arbre est disposé au moins partiellement dans le premier arbre d'entraînement (5, 5'), **caractérisée en ce que** le deuxième arbre (6, 6') est supporté avec possibilité de rotation libre par rapport au premier arbre d'entraînement (5, 5') au moyen d'au moins un palier (7, 7'), de sorte que le deuxième arbre (6, 6') peut tourner indépendamment du premier arbre d'entraînement (5, 5').

2. Poignée (1, 1') à usage médical, en particulier chirurgical, selon la revendication 1, **caractérisée en ce que** le premier arbre d'entraînement (5, 5') est réalisé en plusieurs parties, de préférence en deux parties, sous la forme d'un arbre moteur et d'un premier arbre de sortie (8).

3. Poignée (1, 1') à usage médical, en particulier chirurgical, selon la revendication 1 ou 2, **caractérisée en ce que** la chaîne de transmission (3, 3') présente en outre un engrenage (9, 9') avec un deuxième arbre de sortie (10, 10'), l'engrenage étant relié au premier arbre d'entraînement (5, 5') afin d'entraîner le deuxième arbre de sortie (10, 10') à une vitesse de rotation différente de celle du premier arbre de sortie (5, 5').

4. Poignée (1, 1') à usage médical, en particulier chirurgical, selon la revendication 2 ou 3, **caractérisée en ce que** le deuxième arbre (6, 6') s'étend au moins partiellement à travers le deuxième arbre de sortie (10, 10').

5. Poignée (1, 1') à usage médical, en particulier chirurgical, selon l'une des revendications précédentes, **caractérisée en ce que** l'au moins un palier (7, 7') servant à supporter le deuxième arbre (6, 6') est logé dans la chaîne de transmission.

6. Poignée (1, 1') à usage médical, en particulier chirurgical, selon la revendication 5, **caractérisée en ce que** le palier (7, 7') servant à supporter le deuxième arbre (6, 6') s'appuie sur une extrémité de la chaîne de transmission ou d'une partie d'arbre de la chaîne de transmission, de préférence au moyen d'un épaulement dans la paroi de l'arbre.

7. Poignée (1, 1') à usage médical, en particulier chirurgical, selon l'une des revendications précédente, **caractérisée en ce que** l'au moins un palier (11, 11') servant à supporter le deuxième arbre (6, 6') est supporté sur un composant fixe en rotation de la poignée (1, 1').

8. Poignée (1, 1') à usage médical, en particulier chirurgical, selon la revendication 7, **caractérisée en ce que** le palier (11, 11') servant à supporter le deuxième arbre (6, 6') est reçu dans une ouverture (12, 12') du corps disposée de façon coaxiale de la chaîne de transmission.

9. Poignée (1, 1') à usage médical, en particulier chirurgical, selon l'une des revendications précédente, **caractérisée en ce que** l'au moins un palier (7, 7' ; 11, 11') est un roulement, en particulier un roulement à billes.

10. Poignée (1, 1') à usage médical, en particulier chirurgical, selon l'une des revendications précédentes, **caractérisée en ce que** le dispositif de couplage (13) présente un deuxième engrenage.

11. Poignée (1, 1') à usage médical, en particulier chirurgical, selon l'une des revendications précédentes, caractérisée en ce le deuxième arbre (6, 6') s'étend au moins partiellement à travers l'arbre de couplage (13).

12. Poignée (1, 1') à usage médical, en particulier chirurgical, selon l'une des revendications précédentes, **caractérisée en ce que** le deuxième arbre (6, 6') est réalisé en plusieurs parties, de préférence en deux parties, pour s'appuyer dans l'arbre de sortie (8 ; 10, 10') et dans l'arbre d'accouplement.

13. Poignée (1, 1') à usage médical, en particulier chirurgical, selon l'une des revendications précédentes, **caractérisée en ce que** la chaîne de transmission (3, 3') et le deuxième arbre (6, 6') présentent un alésage traversant (14, 14') qui traverse la poignée.
